# EUROPÄISCHE PATENTSCHRIFT

(11) **EP 1 123 388 B1**
(45) Veröffentlichungstag und Bekanntmachung des Hinweises auf die Patenterteilung: **18.01.2012**
(21) Anmeldenummer: 99947485.1
(22) Anmeldetag: 09.10.1999
(51) Int. Cl.: C12N 15/00, A01K 67/033, C07K 14/47, C12N 15/62, C12N 5/10, A61K 49/00

(54) **TRANSGENER C. ELEGANS ALS MODELLORGANISMUS FÜR UNTERSUCHUNGEN ZUR ALZHEIMER'SCHEN KRANKHEIT**
TRANSGENIC C. ELEGANS AS A MODEL ORGANISM FOR RESEARCH INTO ALZHEIMER'S DISEASE
C. ELEGANS TRANSGENIQUE EN TANT QU'ORGANISME MODELE DESTINE A DES RECHERCHES SUR LA MALADIE D'ALZHEIMER

(30) Priorität: 24.10.1998 DE 19849073
(43) Veröffentlichungstag der Anmeldung: 16.08.2001
(73) Patentinhaber: Sanofi-Aventis Deutschland GmbH, 65929 Frankfurt am Main (DE)
(72) Erfinder: PERAUS, Giesela, D-80687 München (DE); HOPPE, Edmund, D-82152 Krailing (DE); BAUMEISTER, Ralf, D-82194 Gröbenzell (DE)
(86) Internationale Anmeldenummer: PCT/EP1999/007578
(87) Internationale Veröffentlichungsnummer: WO 2000/024880

(56) Entgegenhaltungen:
- WO-A-98/16627
- WO-A-98/28971
- LINK C D: "EXPRESSION OF HUMAN BETA-AMYLOID PEPTIDE IN TRANSGENIC CAENORHABDITIS ELEGANS" PROCEEDINGS OF THE NATIONAL ACADEMY OF SCIENCES OF USA,US,NATIONAL ACADEMY OF SCIENCE. WASHINGTON, Bd. 92, Nr. 20, 1. September 1995 (1995-09-01), Seiten 9368-9372, XP002065639 ISSN: 0027-8424 in der Anmeldung erwähnt
- LINK C D: "TRANSGENIC CAENORHABDITIS ELEGANS AS MODEL SYSTEM TO STUDY AMYLOID FORMATION AND TOXICITY" NEUROBIOLOGY OF AGING,US,TARRYTOWN, NY, Bd. 15, Nr. SUPPL. 01, 29. Juli 1994 (1994-07-29), Seite S33 XP002065640 ISSN: 0197-4580
- KAMMESHEIDT A ET AL: "DEPOSITION OF BETA/A4 IMMUNOREACTIVITY AND NEURONAL PATHOLOGY IN TRANSGENIC MICE EXPRESSING THE CARBOXYL-TERMINAL FRAGMENT OF THE ALZHEIMER AMYLOID PRECURSOR IN THE BRAIN" PROCEEDINGS OF THE NATIONAL ACADEMY OF SCIENCES OF USA,US,NATIONAL ACADEMY OF SCIENCE. WASHINGTON, Bd. 89, Nr. 22, 15. November 1992 (1992-11-15), Seiten 10857-10861, XP000616220 ISSN: 0027-8424
- NEVE, R.L. ET AL.: "Construction and analysis of transgenic mice expressing amyloidogenic fragments of Alzheimer Amyloid Protein Precursor" METHODS IN NEUROSCIENCE, Bd. 30, 1996, Seiten 298-314, XP000870339
- BAUMEISTER R, LEIMER U, ZWECKBRONNER I, JAKUBEK C, GRUNBERG J, HAASS C. : "Human presenilin-1, but not familial Alzheimer's disease (FAD) mutants, facilitate Caenorhabditis elegans Notch signalling independently of proteolytic processing." GENES FUNCT. , Bd. 1, Nr. 2, April 1997 (1997-04), Seiten 149-159, XP000874639 in der Anmeldung erwähnt
- FAY, D.S. ET AL.: "In vivo aggregation of Beta amyloid peptide variants" JOURNAL OF NEUROCHEMISTRY, Bd. 71, Nr. 4, 1. Oktober 1998 (1998-10-01), Seiten 1616-1625, XP000879286
- PERAUS GC, MASTERS CL, BEYREUTHER K.: "Late compartments of amyloid precursor protein transport in SY5Y cells are involved in beta-amyloid secretion." J NEUROSCI., Bd. 17, Nr. 20, 15. Oktober 1997 (1997-10-15), Seiten 7714-7724, XP000870189

### SEQUENZPROTOKOLL

(1) ALLGEMEINE ANGABEN:
   (i) ANMELDER:
      (A) NAME: Hoechst Marion Roussel Deutschland GmbH
      (B) STRASSE: -
      (C) ORT: Frankfurt
      (D) BUNDESLAND: -
      (E) LAND: Deutschland
      (F) POSTLEITZAHL: 65926
      (G) TELEFON: 069-305-7072
      (H) TELEFAX: 069-35-7175
      (I) TELEX: -
   (ii) BEZEICHNUNG DER ERFINDUNG: Transgener C. elegans als Modellorganismus für Untersuchungen zur Alzheimer'schen Krankheit
   (iii) ANZAHL DER SEQUENZEN: 11
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 303 Basenpaare
      (B) ART: Nucleotid
      (C) STRANGFORM: Einzelstrang
      (D) TOPOLOGIE: linear
   (ii) ART DES MOLEKÜLS: Genom-DNA
   (ix) MERKMAL:
      (A) NAME/SCHLÜSSEL: exon
      (B) LAGE:1..303
   (xi) SEQUENZBESCHREIBUNG: SEQ ID NO: 1:
   CTGGATGCAG AATTCCGACA TGACTCAGGA TATGAAGTTC ATCATCAAAA ATTGGTGTTC      60
   TTTGCAGAAG ATGTGGGTTC AAACAAAGGT GCAATCATTG GACTCATGGT GGGCGGTGTT     120
   GTCATAGCGA CAGTGATCGT CATCACCTTG GTGATGCTGA AGAAGAAACA GTACACATCC     180
   ATTCATCATG GTGTGGTGGA GGTTGACGCC GCTGTCACCC CAGAGGAGCG CCACCTGTCC     240
   AAGATGCAGC AGAACGGCTA CGAAAATCCA ACCTACAAGT TCTTTGAGCA GATGCAGAAC     300
   TAG                                                                   303
(2) ANGABEN ZU SEQ ID NO: 2:
   (i) SEQUENZKENNZEICHEN:
      (A) LÄNGE: 51 Basenpaare
      (B) ART: Nucleotid
      (C) STRANGFORM: Einzelstrang
      (D) TOPOLOGIE: linear
   (ii) ART DES MOLEKÜLS: Genom-DNA
   (ix) MERKMAL:
      (A) NAME/SCHLÜSSEL: exon
      (B) LAGE:1..51
   (xi) SEQUENZBESCHREIBUNG: SEQ ID NO: 2:
      ATGCTGCCCG GTTTGGCACT GTTCCTGCTG GCCGCCTGGA CGGCTCGGGC G 51
(2) ANGABEN ZU SEQ ID NO: 3:
   (i) SEQUENZKENNZEICHEN:
      (A) LÄNGE: 354 Basenpaare
      (B) ART: Nucleotid
      (C) STRANGFORM: Einzelstrang
      (D) TOPOLOGIE: linear
   (ii) ART DES MOLEKÜLS: Genom-DNA
   (ix) MERKMAL:
      (A) NAME/SCHLÜSSEL: exon
      (B) LAGE:1..354 (xi) SEQUENZBESCHREIBUNG: SEQ ID NO: 3:
(2) ANGABEN ZU SEQ ID NO: 4:
   (i) SEQUENZKENNZEICHEN:
      (A) LÄNGE: 42 Basenpaare
      (B) ART: Nucleotid
      (C) STRANGFORM: Einzelstrang
      (D) TOPOLOGIE: linear
   (ii) ART DES MOLEKÜLS: Genom-DNA (ix) MERKMAL:
      (A) NAME/SCHLÜSSEL: exon
      (B) LAGE:1..42
   (xi) SEQUENZBESCHREIBUNG: SEQ ID NO: 4:
      GTATGTTTCG AATGATACTA ACATAACATA GAACATTTTC AG 42
(2) ANGABEN ZU SEQ ID NO: 5:
   (i) SEQUENZKENNZEICHEN:
      (A) LÄNGE: 495 Basenpaare
      (B) ART: Nucleotid
      (C) STRANGFORM: Einzelstrang
      (D) TOPOLOGIE: linear
   (ii) ART DES MOLEKÜLS: Genom-DNA
   (ix) MERKMAL:
      (A) NAME/SCHLÜSSEL: exon
      (B) LAGE:1..495 (xi) SEQUENZBESCHREIBUNG: SEQ ID NO: 5:
(2) ANGABEN ZU SEQ ID NO: 6:
   (i) SEQUENZKENNZEICHEN:
      (A) LÄNGE: 1207 Basenpaare
      (B) ART: Nucleotid
      (C) STRANGFORM: Einzelstrang
      (D) TOPOLOGIE: linear
   (ii) ART DES MOLEKÜLS: Genom-DNA
   (ix) MERKMAL:
      (A) NAME/SCHLÜSSEL: exon
      (B) LAGE:1..1207
   (xi) SEQUENZBESCHREIBUNG: SEQ ID NO: 6:
   AAGCTTCAGT AAAAGAAGTA GAATTTTATA GTTTTTTTTC TGTTTGAAAA ATTCTCCCCA      60
   TCAATGTTCT TTCAAATAAA TACATCACTA ATGCAAAGTA TTCTATAACC TCATATCTAA     120
   ATTCTTCAAA ATCTTAACAT ATCTTATCAT TGCTTTAAGT CAACGTAACA TTAAAAAAAA     180
   TGTTTTGGAA AATGTGTCAA GTCTCTCAAA ATTCAGTTTT TTAAACCACT CCTATAGTCC     240
   TATAGTCCTA TAGTTACCCA TGAAATCCTT ATATATTACT GTAAAATGTT TCAAAAACCA     300
   TTGGCAAATT GCCAGAACTG AAAATTTCCG GCAAATTGGG GAACCGGCAA ATTGCCAATT     360
   TGCTGAATTT GCCGGAAACG GTAATTGCCG AAAGTTTTTG ACACGAAAAT GGCAAATTGT     420
   GGTTTTAAAA TTTTTTTTTT TGGAAATTTC AGAATTTCAA TTTTAATCGG CAAAACTGTA     480
   GGCATCCTAA GAATGTTCCT ACATCTATTT TGAAAAGTAA GCGAATTAAT TCTATGAAAA     540
   TGTCTAAAGA AAATGGGGAA ACAATTTCAA AAAGGCACAG TTTCAATGGT TTCCGAATTA     600
   TACTAAATCC CTCTAAAAAC TTCCGGCAAA TTGATATCCG TAAAAGAGCA AATCCGCATT     660
   TTTGCCGAAA ATTAAAATTT CCGACAAATC GGCAAACCGG CAATTTGGCG AAATTTGCCG     720
   GAACGATTGC CGCCCACCCC TGTTCCAGAG GTTCAAACTG GTAGCAAAGC TCAAAATTTC     780
   TCAAATTCTC CAATTTTTTT TTGAATTTTG GCAGTGTACC AAAATGACAT TCAGTCATAT     840
   TGGTTTATTA TAGATTTATT TAGATAAAAT CCTAAATGAT TCTACCTTTA AAGATGCCCA     900
   CTTTAAAAGT AATGACTCAA ACTTCAAATT GCTCTAAGAT TCTATTGAAT TACCATCTTT     960
   TCCTCTCATT TTCTCTCACT GTCTATTTCA TCACAAATTC ATCCCTCTCT CCTCTCTTCT    1020
   CTCTCCCTCT CTCTCTCTTT CTCTTTGCTC ATCATCTGTC ATTTTGTCCG TTCCTCTCTC    1080
   TGCGCCCTCA GCGTTCCCCA CACTCTCTCG CTTCTCTTTT CCTAGACGTC TTCTTTTTTC    1140
   ATCTTCTTCA GCCTTTTTCG CCATTTTCCA TCTCTGTCAA TCATTACGGA CGACCCCCAT    1200
   TATCGAT                                                              1207
(2) ANGABEN ZU SEQ ID NO: 7:
   (i) SEQUENZKENNZEICHEN:
      (A) LÄNGE: 1773 Basenpaare
      (B) ART: Nucleotid
      (C) STRANGFORM: Einzelstrang
      (D) TOPOLOGIE: linear
   (ii) ART DES MOLEKÜLS: Genom-DNA
   (ix) MERKMAL:
      (A) NAME/SCHLÜSSEL: exon
      (B) LAGE:1..1773
   (xi) SEQUENZBESCHREIBUNG: SEQ ID NO: 7:
   AAGCTTCAGT AAAAGAAGTA GAATTTTATA GTTTTTTTTC TGTTTGAAAA ATTCTCCCCA     60
   TCAATGTTCT TTCAAATAAA TACATCACTA ATGCAAAGTA TTCTATAACC TCATATCTAA    120
   ATTCTTCAAA ATCTTAACAT ATCTTATCAT TGCTTTAAGT CAACGTAACA TTAAAAAAAA    180
   TGTTTTGGAA AATGTGTCAA GTCTCTCAAA ATTCAGTTTT TTAAACCACT CCTATAGTCC    240
   TATAGTCCTA TAGTTACCCA TGAAATCCTT ATATATTACT GTAAAATGTT TCAAAAACCA    300
   TTGGCAAATT GCCAGAACTG AAAATTTCCG GCAAATTGGG GAACCGGCAA ATTGCCAATT    360
   TGCTGAATTT GCCGGAAACG GTAATTGCCG AAAGTTTTTG ACACGAAAAT GGCAAATTGT    420
   GGTTTTAAAA TTTTTTTTTT TGGAAATTTC AGAATTTCAA TTTTAATCGG CAAAACTGTA    480
   GGCATCCTAA GAATGTTCCT ACATCTATTT TGAAAAGTAA GCGAATTAAT TCTATGAAAA    540
   TGTCTAAAGA AAATGGGGAA ACAATTTCAA AAAGGCACAG TTTCAATGGT TTCCGAATTA    600
   TACTAAATCC CTCTAAAAAC TTCCGGCAAA TTGATATCCG TAAAAGAGCA AATCCGCATT    660
   TTTGCCGAAA ATTAAAATTT CCGACAAATC GGCAAACCGG CAATTTGGCG AAATTTGCCG    720
   GAACGATTGC CGCCCACCCC TGTTCCAGAG GTTCAAACTG GTAGCAAAGC TCAAAATTTC    780
   TCAAATTCTC CAATTTTTTT TTGAATTTTG GCAGTGTACC AAAATGACAT TCAGTCATAT    840
   TGGTTTATTA TAGATTTATT TAGATAAAAT CCTAAATGAT TCTACCTTTA AAGATGCCCA    900
   CTTTAAAAGT AATGACTCAA ACTTCAAATT GCTCTAAGAT TCTATTGAAT TACCATCTTT    960
   TCCTCTCATT TTCTCTCACT GTCTATTTCA TCACAAATTC ATCCCTCTCT CCTCTCTTCT   1020
   CTCTCCCTCT CTCTCTCTTT CTCTTTGCTC ATCATCTGTC ATTTTGTCCG TTCCTCTCTC   1080
   TGCGCCCTCA GCGTTCCCCA CACTCTCTCG CTTCTCTTTT CCTAGACGTC TTCTTTTTTC   1140
   ATCTTCTTCA GCCTTTTTCG CCATTTTCCA TCTCTGTCAA TCATTACGGA CGACCCCCAT   1200
   TATCGATAAG ATCTCCACGG TGGCCGCGAA TTCCTGCAGC CCGGGGGATC CCCGGGATTG   1260
   GCCAAAGGAC CCAAAGGTAT GTTTCGAATG ATACTAACAT AACATAGAAC ATTTTCAGGA   1320
   GGACCCTTGG CTAGCGTCGA CGGTACCGGG CCCCCCCTCG AGGTCGACGG TATCGATAAC   1380
   CTTCACAGCA GCGCACTCGG TGCCCCGCGC AGGGTCGCGA TGCTGCCCGG TTTGGCACTG   1440
   TTCCTGCTGG CCGCCTGGAC GGCTCGGGCG CTGGATGCAG AATTCCGACA TGACTCAGGA   1500
   TATGAAGTTC ATCATCAAAA ATTGGTGTTC TTTGCAGAAG ATGTGGGTTC AAACAAAGGT   1560
   GCAATCATTG GACTCATGGT GGGCGGTGTT GTCATAGCGA CAGTGATCGT CATCACCTTG   1620
   GTGATGCTGA AGAAGAAACA GTACACATCC ATTCATCATG GTGTGGTGGA GGTTGACGCC 1680
   GCTGTCACCC CAGAGGAGCG CCACCTGTCC AAGATGCAGC AGAACGGCTA CGAAAATCCA 1740
   ACCTACAAGT TCTTTGAGCA GATGCAGAAC TAG 1773
(2) ANGABEN ZU SEQ ID NO: 8:
   (i) SEQUENZKENNZEICHEN:
      (A) LÄNGE: 3344 Basenpaare
      (B) ART: Nucleotid
      (C) STRANGFORM: Einzelstrang
      (D) TOPOLOGIE: linear
   (ii) ART DES MOLEKÜLS: Genom-DNA
   (ix) MERKMAL:
      (A) NAME/SCHLÜSSEL: exon
      (B) LAGE:1..3344
   (xi) SEQUENZBESCHREIBUNG: SEQ ID NO: 8:
   ACCCCCGCCA CAGCAGCCTC TGAAGTTGGA CACGGATCCA CTAGTTCTAG AGCGGCCGCC 60
   ACCGCGGTGG AGCTCCGCAT CGGCCGCTGT CATCAGATCG CCATCTCGCG CCCGTGCCTC 120
   TGACTTCTAA GTCCAATTAC TCTTCAACAT CCCTACATGC TCTTTCTCCC TGTGCTCCCA 180
   CCCCCTATTT TTGTTATTAT CAAAAAAACT TCTTCTTAAT TTCTTTGTTT TTTAGCTTCT 240
   TTTAAGTCAC CTCTAACAAT GAAATTGTGT AGATTCAAAA ATAGAATTAA TTCGTAATAA 300
   AAAGTCGAAA AAAATTGTGC TCCCTCCCCC CATTAATAAT AATTCTATCC CAAAATCTAC 360
   ACAATGTTCT GTGTACACTT CTTATGTTTT TTTTACTTCT GATAAATTTT TTTTGAAACA 420
   TCATAGAAAA AACCGCACAC AAAATACCTT ATCATATGTT ACGTTTCAGT TTATGACCGC 480
   AATTTTTATT TCTTCGCACG TCTGGGCCTC TCATGACGTC AAATCATGCT CATCGTGAAA 540
   AAGTTTTGGA GTATTTTTGG AATTTTTCAA TCAAGTGAAA GTTTATGAAA TTAATTTTCC 600
   TGCTTTTGCT TTTTGGGGGT TTCCCCTATT GTTTGTCAAG AGTTTCGAGG ACGGCGTTTT 660
   TCTTGCTAAA ATCACAAGTA TTGATGAGCA CGATGCAAGA AAGATCGGAA GAAGGTTTGG 720
   GTTTGAGGCT CAGTGGAAGG TGAGTAGAAG TTGATAATTT GAAAGTGGAG TAGTGTCTAT 780
   GGGGTTTTTG CCTTAAATGA CAGAATACAT TCCCAATATA CCAAACATAA CTGTTTCCTA 840
   CTAGTCGGCC GTACGGGCCC TTTCGTCTCG CGCGTTTCGG TGATGACGGT GAAAACCTCT 900
   GACACATGCA GCTCCCGGAG ACGGTCACAG CTTGTCTGTA AGCGGATGCC GGGAGCAGAC 960
   AAGCCCGTCA GGGCGCGTCA GCGGGTGTTG GCGGGTGTCG GGGCTGGCTT AACTATGCGG 1020
   CATCAGAGCA GATTGTACTG AGAGTGCACC ATATGCGGTG TGAAATACCG CACAGATGCG 1080
   TAAGGAGAAA ATACCGCATC AGGCGGCCTT AAGGGCCTCG TGATACGCCT ATTTTTATAG 1140
   GTTAATGTCA TGATAATAAT GGTTTCTTAG ACGTCAGGTG GCACTTTTCG GGGAAATGTG 1200
   CGCGGAACCC CTATTTGTTT ATTTTTCTAA ATACATTCAA ATATGTATCC GCTCATGAGA 1260
   CAATAACCCT GATAAATGCT TCAATAATAT TGAAAAAGGA AGAGTATGAG TATTCAACAT 1320
   TTCCGTGTCG CCCTTATTCC CTTTTTTGCG GCATTTTGCC TTCCTGTTTT TGCTCACCCA 1380
   GAAACGCTGG TGAAAGTAAA AGATGCTGAA GATCAGTTGG GTGCACGAGT GGGTTACATC 1440
   GAACTGGATC TCAACAGCGG TAAGATCCTT GAGAGTTTTC GCCCCGAAGA ACGTTTTCCA 1500
   ATGATGAGCA CTTTTAAAGT TCTGCTATGT GGCGCGGTAT TATCCCGTAT TGACGCCGGG 1560
   CAAGAGCAAC TCGGTCGCCG CATACACTAT TCTCAGAATG ACTTGGTTGA GTACTCACCA 1620
   GTCACAGAAA AGCATCTTAC GGATGGCATG ACAGTAAGAG AATTATGCAG TGCTGCCATA 1680
   ACCATGAGTG ATAACACTGC GGCCAACTTA CTTCTGACAA CGATCGGAGG ACCGAAGGAG 1740
   CTAACCGCTT TTTTGCACAA CATGGGGGAT CATGTAACTC GCCTTGATCG TTGGGAACCG 1800
   GAGCTGAATG AAGCCATACC AAACGACGAG CGTGACACCA CGATGCCTGT AGCAATGGCA 1860
   ACAACGTTGC GCAAACTATT AACTGGCGAA CTACTTACTC TAGCTTCCCG GCAACAATTA 1920
   ATAGACTGGA TGGAGGCGGA TAAAGTTGCA GGACCACTTC TGCGCTCGGC CCTTCCGGCT 1980
   GGCTGGTTTA TTGCTGATAA ATCTGGAGCC GGTGAGCGTG GGTCTCGCGG TATCATTGCA 2040
   GCACTGGGCC AGATGGTAAG CCCTCCCGTA TCGTAGTTAT CTACACGACG GGGAGTCAGG 2100
   CAACTATGGA TGAACGAAAT AGACAGATCG CTGAGATAGG TGCCTCACTG ATTAAGCATT 2160
   GGTAACTGTC AGACCAAGTT TACTCATATA TACTTTAGAT TGATTTAAAA CTTCATTTTT 2220
   AATTTAAAAG GATCTAGGTG AAGATCCTTT TTGATAATCT CATGACCAAA ATCCCTTAAC 2280
   GTGAGTTTTC GTTCCACTGA GCGTCAGACC CCGTAGAAAA GATCAAAGGA TCTTCTTGAG 2340
   ATCCTTTTTT TCTGCGCGTA ATCTGCTGCT TGCAAACAAA AAAACCACCG CTACCAGCGG 2400
   TGGTTTGTTT GCCGGATCAA GAGCTACCAA CTCTTTTTCC GAAGGTAACT GGCTTCAGCA 2460
   GAGCGCAGAT ACCAAATACT GTCCTTCTAG TGTAGCCGTA GTTAGGCCAC CACTTCAAGA 2520
   ACTCTGTAGC ACCGCCTACA TACCTCGCTC TGCTAATCCT GTTACCAGTG GCTGCTGCCA 2580
   GTGGCGATAA GTCGTGTCTT ACCGGGTTGG ACTCAAGACG ATAGTTACCG GATAAGGCGC 2640
   AGCGGTCGGG CTGAACGGGG GGTTCGTGCA CACAGCCCAG CTTGGAGCGA ACGACCTACA 2700
   CCGAACTGAG ATACCTACAG CGTGAGCATT GAGAAAGCGC CACGCTTCCC GAAGGGAGAA 2760
   AGGCGGACAG GTATCCGGTA AGCGGCAGGG TCGGAACAGG AGAGCGCACG AGGGAGCTTC 2820
   CAGGGGGAAA CGCCTGGTAT CTTTATAGTC CTGTCGGGTT TCGCCACCTC TGACTTGAGC 2880
   GTCGATTTTT GTGATGCTCG TCAGGGGGGC GGAGCCTATG GAAAAACGCC AGCAACGCGG 2940
   CCTTTTTACG GTTCCTGGCC TTTTGCTGGC CTTTTGCTCA CATGTTCTTT CCTGCGTTAT 3000
   CCCCTGATTC TGTGGATAAC CGTATTACCG CCTTTGAGTG AGCTGATACC GCTCGCCGCA 3060
   GCCGAACGAC CGAGCGCAGC GAGTCAGTGA GCGAGGAAGC GGAAGAGCGC CCAATACGCA 3120
   AACCGCCTCT CCCCGCGCGT TGGCCGATTC ATTAATGCAG CTGGCACGAC AGGTTTCCCG 3180
   ACTGGAAAGC GGGCAGTGAG CGCAACGCAA TTAATGTGAG TTAGCTCACT CATTAGGCAC 3240
   CCCAGGCTTT ACACTTTATG CTTCCGGCTC GTATGTTGTG TGGAATTGTG AGCGGATAAC 3300
   AATTTCACAC AGGAAACAGC TATGACCATG ATTACGCCAA GCTT 3344
(2) ANGABEN ZU SEQ ID NO: 9:
   (i) SEQUENZKENNZEICHEN:
      (A) LÄNGE: 17 Aminosäuren
      (B) ART: Aminosäure
      (C) STRANGFORM: Einzelstrang
      (D) TOPOLOGIE: linear
   (ii) ART DES MOLEKÜLS: Protein
   (ix) MERKMAL:
      (A) NAME/SCHLÜSSEL: Protein
      (B) LAGE:1..17
   (xi) SEQUENZBESCHREIBUNG: SEQ ID NO: 9:
   Met Leu Pro Gly Leu Ala Leu Phe Leu Leu Ala Ala Trp Thr Ala Arg
   1 5 10 15
   Ala
(2) ANGABEN ZU SEQ ID NO: 10:
   (i) SEQUENZKENNZEICHEN:
      (A) LÄNGE: 117 Aminosäuren
      (B) ART: Aminosäure
      (C) STRANGFORM: Einzelstrang
      (D) TOPOLOGIE: linear
   (ii) ART DES MOLEKÜLS: Protein
   (ix) MERKMAL:
      (A) NAME/SCHLÜSSEL: Protein
      (B) LAGE:1..117 (xi) SEQUENZBESCHREIBUNG: SEQ ID NO: 10:
(2) ANGABEN ZU SEQ ID NO: 11:
   (i) SEQUENZKENNZEICHEN:
      (A) LÄNGE: 5109 Basenpaare
      (B) ART: Nucleotid
      (C) STRANGFORM: Einzelstrang
      (D) TOPOLOGIE: linear
   (ii) ART DES MOLEKÜLS: Genom-DNA
   (ix) MERKMAL:
      (A) NAME/SCHLÜSSEL: exon
      (B) LAGE:1..5109
   (xi) SEQUENZBESCHREIBUNG: SEQ ID NO: 11:
   ATGACCATGA TTACGCCAAG CTTCAGTAAA AGAAGTAGAA TTTTATAGTT TTTTTTCTGT 60
   TTGAAAAATT CTCCCCATCA ATGTTCTTTC AAATAAATAC ATCACTAATG CAAAGTATTC 120
   TATAACCTCA TATCTAAATT CTTCAAAATC TTAACATATC TTATCATTGC TTTAAGTCAA 180
   CGTAACATTA AAAAAAATGT TTTGGAAAAT GTGTCAAGTC TCTCAAAATT CAGTTTTTTA 240
   AACCACTCCT ATAGTCCTAT AGTCCTATAG TTACCCATGA AATCCTTATA TATTACTGTA 300
   AAATGTTTCA AAAACCATTG GCAAATTGCC AGAACTGAAA ATTTCCGGCA AATTGGGGAA 360
   CCGGCAAATT GCCAATTTGC TGAATTTGCC GGAAACGGTA ATTGCCGAAA GTTTTTGACA 420
   CGAAAATGGC AAATTGTGGT TTTAAAATTT TTTTTTTTGG AAATTTCAGA ATTTCAATTT 480
   TAATCGGCAA AACTGTAGGC ATCCTAAGAA TGTTCCTACA TCTATTTTGA AAAGTAAGCG 540
   AATTAATTCT ATGAAAATGT CTAAAGAAAA TGGGGAAACA ATTTCAAAAA GGCACAGTTT 600
   CAATGGTTTC CGAATTATAC TAAATCCCTC TAAAAACTTC CGGCAAATTG ATATCCGTAA 660
   AAGAGCAAAT CCGCATTTTT GCCGAAAATT AAAATTTCCG ACAAATCGGC AAACCGGCAA 720
   TTTGGCGAAA TTTGCCGGAA CGATTGCCGC CCACCCCTGT TCCAGAGGTT CAAACTGGTA 780
   GCAAAGCTCA AAATTTCTCA AATTCTCCAA TTTTTTTTTG AATTTTGGCA GTGTACCAAA 840
   ATGACATTCA GTCATATTGG TTTATTATAG ATTTATTTAG ATAAAATCCT AAATGATTCT 900
   ACCTTTAAAG ATGCCCACTT TAAAAGTAAT GACTCAAACT TCAAATTGCT CTAAGATTCT 960
   ATTGAATTAC CATCTTTTCC TCTCATTTTC TCTCACTGTC TATTTCATCA CAAATTCATC 1020
   CCTCTCTCCT CTCTTCTCTC TCCCTCTCTC TCTCTTTCTC TTTGCTCATC ATCTGTCATT 1080
   TTGTCCGTTC CTCTCTCTGC GCCCTCAGCG TTCCCCACAC TCTCTCGCTT CTCTTTTCCT 1140
   AGACGTCTTC TTTTTTCATC TTCTTCAGCC TTTTTCGCCA TTTTCCATCT CTGTCAATCA 1200
   TTACGGACGA CCCCCATTAT CGATAAGATC TCCACGGTGG CCGCGAATTC CTGCAGCCCG 1260
   GGGGATCCCC GGGATTGGCC AAAGGACCCA AAGGTATGTT TCGAATGATA CTAACATAAC 1320
   ATAGAACATT TTCAGGAGGA CCCTTGGCTA GCGTCGACGG TACCGGGCCC CCCCTCGAGG 1380
   TCGACGGTAT CGATAACCTT CACAGCAGCG CACTCGGTGC CCCGCGCAGG GTCGCGATGC 1440
   TGCCCGGTTT GGCACTGTTC CTGCTGGCCG CCTGGACGGC TCGGGCGCTG GATGCAGAAT 1500
   TCCGACATGA CTCAGGATAT GAAGTTCATC ATCAAAAATT GGTGTTCTTT GCAGAAGATG 1560
   TGGGTTCAAA CAAAGGTGCA ATCATTGGAC TCATGGTGGG CGGTGTTGTC ATAGCGACAG 1620
   TGATCGTCAT CACCTTGGTG ATGCTGAAGA AGAAACAGTA CACATCCATT CATCATGGTG 1680
   TGGTGGAGGT TGACGCCGCT GTCACCCCAG AGGAGCGCCA CCTGTCCAAG ATGCAGCAGA 1740
   ACGGCTACGA AAATCCAACC TACAATTCTT TGAGCAGATG CAGAACTAGA CCCCCGCCAC 1800
   AGCAGCCTCT GAAGTTGGAC ACGGATCCAC TAGTTCTAGA GCGGCCGCCA CCGCGGTGGA 1860
   GCTCCGCATC GGCCGCTGTC ATCAGATCGC CATCTCGCGC CCGTGCCTCT GACTTCTAAG 1920
   TCCAATTACT CTTCAACATC CCTACATGCT CTTTCTCCCT GTGCTCCCAC CCCCTATTTT 1980
   TGTTATTATC AAAAAAACTT CTTCTTAATT TCTTTGTTTT TAGCTTCTTT TAAGTCACCT 2040
   CTAACAATGA AATTGTGTAG ATTCAAAAAT AGAATTAATT CGTAATAAAA AGTCGAAAAA 2100
   AATTGTGCTC CCTCCCCCCA TTAATAATAA TTCTATCCCA AAATCTACAC AATGTTCTGT 2160
   GTACACTTCT TATGTTTTTT TTACTTCTGA TAAATTTTTT TTGAAACATC ATAGAAAAAA 2220
   CCGCACACAA AATACCTTAT CATATGTTAC GTTTCAGTTT ATGACCGCAA TTTTTATTTC 2280
   TTCGCACGTC TGGGCCTCTC ATGACGTCAA ATCATGCTCA TCGTGAAAAA GTTTTGGAGT 2340
   ATTTTTGGAA TTTTTCAATC AAGTGAAAGT TTATGAAATT AATTTTCCTG CTTTTGCTTT 2400
   TTGGGGGTTT CCCCTATTGT TTGTCAAGAG TTTCGAGGAC GGCGTTTTTC TTGCTAAAAT 2460
   CACAAGTATT GATGAGCACG ATGCAAGAAA GATCGGAAGA AGGTTTGGGT TTGAGGCTCA 2520
   GTGGAAGGTG AGTAGAAGTT GATAATTTGA AAGTGGAGTA GTGTCTATGG GGTTTTTGCC 2580
   TTAAATGACA GAATACATTC CCAATATACC AAACATAACT GTTTCCTACT AGTCGGCCGT 2640
   ACGGGCCCTT TCGTCTCGCG CGTTTCGGTG ATGACGGTGA AAACCTCTGA CACATGCAGC 2700
   TCCCGGAGAC GGTCACAGCT TGTCTGTAAG CGGATGCCGG GAGCAGACAA GCCCGTCAGG 2760
   GCGCGTCAGC GGGTGTTGGC GGGTGTCGGG GCTGGCTTAA CTATGCGGCA TCAGAGCAGA 2820
   TTGTACTGAG AGTGCACCAT ATGCGGTGTG AAATACCGCA CAGATGCGTA AGGAGAAAAT 2880
   ACCGCATCAG GCGGCCTTAA GGGCCTCGTG ATACGCCTAT TTTTATAGGT TAATGTCATG 2940
   ATAATAATGG TTTCTTAGAC GTCAGGTGGC ACTTTTCGGG GAAATGTGCG CGGAACCCCT 3000
   ATTTGTTTAT TTTTCTAAAT ACATTCAAAT ATGTATCCGC TCATGAGACA ATAACCCTGA 3060
   TAAATGCTTC AATAATATTG AAAAAGGAAG AGTATGAGTA TTCAACATTT CCGTGTCGCC 3120
   CTTATTCCCT TTTTTGCGGC ATTTTGCCTT CCTGTTTTTG CTCACCCAGA AACGCTGGTG 3180
   AAAGTAAAAG ATGCTGAAGA TCAGTTGGGT GCACGAGTGG GTTACATCGA ACTGGATCTC 3240
   AACAGCGGTA AGATCCTTGA GAGTTTTCGC CCCGAAGAAC GTTTTCCAAT GATGAGCACT 3300
   TTTAAAGTTC TGCTATGTGG CGCGGTATTA TCCCGTATTG ACGCCGGGCA AGAGCAACTC 3360
   GGTCGCCGCA TACACTATTC TCAGAATGAC TTGGTTGAGT ACTCACCAGT CACAGAAAAG 3420
   CATCTTACGG ATGGCATGAC AGTAAGAGAA TTATGCAGTG CTGCCATAAC CATGAGTGAT 3480
   AACACTGCGG CCAACTTACT TCTGACAACG ATCGGAGGAC CGAAGGAGCT AACCGCTTTT 3540
   TTGCACAACA TGGGGGATCA TGTAACTCGC CTTGATCGTT GGGAACCGGA GCTGAATGAA 3600
   GCCATACCAA ACGACGAGCG TGACACCACG ATGCCTGTAG CAATGGCAAC AACGTTGCGC 3660
   AAACTATTAA CTGGCGAACT ACTTACTCTA GCTTCCCGGC AACAATTAAT AGACTGGATG 3720
   GAGGCGGATA AAGTTGCAGG ACCACTTCTG CGCTCGGCCC TTCCGGCTGG CTGGTTTATT 3780
   GCTGATAAAT CTGGAGCCGG TGAGCGTGGG TCTCGCGGTA TCATTGCAGC ACTGGGGCCA 3840
   GATGGTAAGC CCTCCCGTAT CGTAGTTATC TACACGACGG GGAGTCAGGC AACTATGGAT 3900
   GAACGAAATA GACAGATCGC TGAGATAGGT GCCTCACTGA TTAAGCATTG GTAACTGTCA 3960
   GACCAAGTTT ACTCATATAT ACTTTAGATT GATTTAAAAC TTCATTTTTA ATTTAAAAGG 4020
   ATCTAGGTGA AGATCCTTTT TGATAATCTC ATGACCAAAA TCCCTTAACT GAGTTTTCGT 4080
   TCCACTGAGC GTCAGACCCC GTAGAAAAGA TCAAAGGATC TTCTTGAGAT CCTTTTTTTC 4140
   TGCGCGTAAT CTGCTGCTTG CAAACAAAAA AACCACCGCT ACCAGCGGTG GTTTGTTTGC 4200
   CGGATCAAGA GCTACCAACT CTTTTTCCGA AGGTAACTGG CTTCAGCAGA GCGCAGATAC 4260
   CAAATACTGT CCTTCTAGTG TAGCCGTAGT TAGGCCACCA CTTCAAGAAC TCTGTAGCAC 4320
   CGCCTACATA CCTCGCTCTG CTAATCCTGT TACCAGTGGC TGCTGCCAGT GGCGATAAGT 4380
   CGTGTCTTAC CGGGTTGGAC TCAAGACGAT AGTTACCGGA TAAGGCGCAG CGGTCGGGCT 4440
   GAACGGGGGG TTCGTGCACA CAGCCCAGCT TGGAGCGAAC GACCTACACC GAACTGAGAT 4500
   ACCTACAGCG TGAGCATTGA GAAAGCGCCA CGCTTCCCGA AGGGAGAAAG GCGGACAGGT 4560
   ATCCGGTAAG CGGCAGGGTC GGAACAGGAG AGCGCACGAG GGAGCTTCCA GGGGGAAACG 4620
   CCTGGTATCT TTATAGTCCT GTCGGGTTTC GCCACCTCTG ACTTGAGCGT CGATTTTTGT 4680
   GATGCTCGTC AGGGGGGCGG AGCCTATGGA AAAACGCCAG CAACGCGGCC TTTTTACGGT 4740
   TCCTGGCCTT TTGCTGGCCT TTTGCTCACA TGTTCTTTCC TGCGTTATCC CCTGATTCTG 4800
   TGGATAACCG TATTACCGCC TTTGAGTGAG CTGATACCGC TCGCCGCAGC CGAACGACCG 4860
   AGCGCAGCGA GTCAGTGAGC GAGGAAGCGG AAGAGCGCCC AATACGCAAA CCGCCTCTCC 4920
   CCGCGCGTTG GCCGATTCAT TAATGCAGCT GGCACGACAG GTTTCCCGAC TGGAAAGCGG 4980
   GCAGTGAGCG CAACGCAATT AATGTGAGTT AGCTCACTCA TTAGGCACCC CAGGCTTTAC 5040
   ACTTTATGCT TCCGGCTCGT ATGTTGTGTG GAATTGTGAG CGGATAACAA TTTCACACAG 5100
   GAAACAGCT 5109

## Beschreibung

Die vorliegende Erfindung betrifft die Verwendung eines transgenen C. elegans enthaltend ein Transgen, welches für da Amyloid Vorläufer Protein (APP) oder einen Teil davon kodiert, zur Identifizierung und/oder Charakterisierung von Substanzen, die die γ-Sekretase Aktivität hemmen oder die die α-Sekretase Aktivität erhöhen, bzw. zur Validierung einer α- und/oder einer γ-Sekretionsaktivität bzw. zur Identifizierung einer γ-Sekretase Aktivität in C. elegans.

Bei der Alzheimer'schen Krankheit (Morbus Alzheimer) handelt es sich um eine neurodegenerative Erkrankung des Gehirns, die auf zellulärer Ebene mit einem massiven Verlust von Neuronen im limbischen System und im cerebralen Cortex einher geht. Auf molekularer Ebene lassen sich in den betroffenen Gehirnarealen Proteinablagerungen, sogenannte Plaques, nachweisen, die ein wesentliches Charakteristikum der Alzheimer'schen Krankheit darstellen. Das am häufigsten in diesen Plaques vorkommende Protein ist ein 40 bis 42 Aminosäuren großes Peptid, das als Aß-Peptid bezeichnet wird. Bei diesem Peptid handelt es sich um ein Spaltprodukt eines wesentlich größeren Proteins von 695 bis 751 Aminosäuren, dem sogenannten Amyloidvorläuferprotein (Amyloid Precursor Protein; APP).

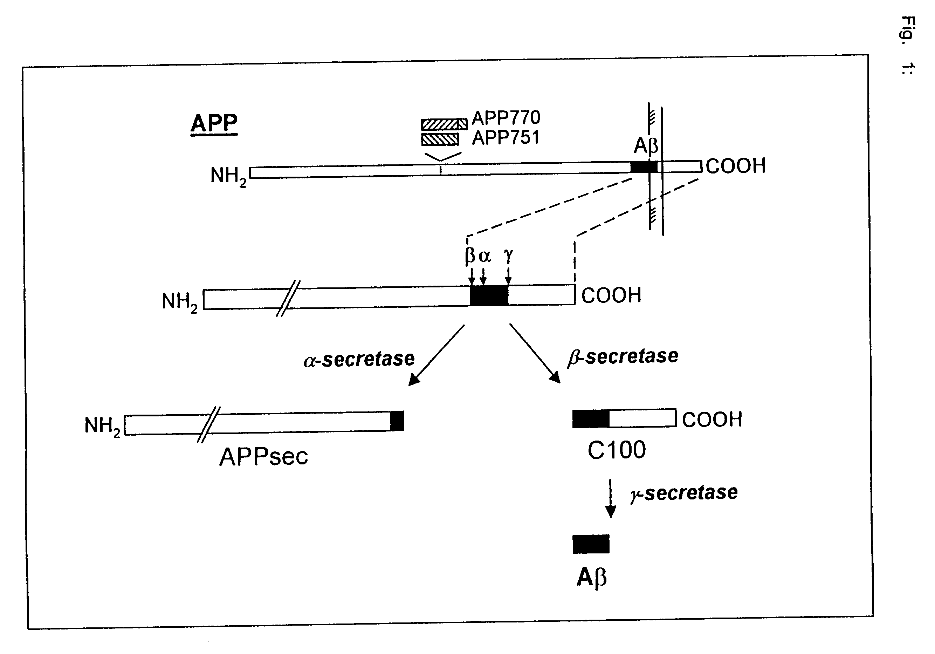

APP ist ein integrales Transmembranprotein, das die Lipiddoppelschicht einmal durchquert. Der weitaus größte Teil des Proteins liegt extrazellulär, während die kürzere C-terminale Domäne in das Cytosol gerichtet ist (Figur 1). Das Aß-Peptid ist in Figur 1 dunkelgrau dargestellt. Etwa zwei Drittel des Aß-Peptids stammen aus der extrazellulären Domäne und etwa ein Drittel aus der Transmembrandomäne von APP.

Neben dem membranständigen APP läßt sich eine sekretierte Form des Amyloid Vorläuferproteins nachweisen, die aus der großen Ektodomäne des APPs besteht und als APPsec ("sekretiertes APP") bezeichnet wird. APPsec entsteht aus APP durch proteolytische Spaltung, die durch die ∀-Sekretase erfolgt. Die proteolytische

Spaltung findet an einer Stelle der Aminosäuresequenz von APP statt, die innerhalb der Aminosäuresequenz des Aß-Peptids liegt (nach Aminosäurerest 16 des Aß-Peptids). Eine Proteolyse von APP durch die α-Sekretase schließt damit die Bildung des Aß-Peptids aus.

Das Aß-Peptid kann also nur auf einem alternativen Prozessierungsweg aus APP gebildet werden. Es wird postuliert, daß an diesem Prozessierungsweg zwei weitere Proteasen beteiligt sind, wobei die eine Protease, die als ß-Sekretase bezeichnet wird, am N-Terminus des Aß-Peptids im APP schneidet und die zweite Protease, die als γ-Sekretase bezeichnet wird, den C-Terminus des Aß-Peptids freisetzt (Kang, J. et al., Nature, 325, 733) (Figur 1).

Bisher konnte keine der drei Sekretasen bzw. Proteasen (α-Sekretase, β-Sekretase, γ-Sekretase) identifiziert werden. Die Kenntnis der Sekretasen ist jedoch von großem Interesse, insbesondere im Rahmen von Untersuchungen zur Alzheimer'schen Krankheit und zur Identifizierung der beteiligten Proteine, die dann wiederum als Targets in weiterführenden Studien eingesetzt werden können, da zum einen die Inhibition der ß - und insbesondere der γ-Sekretase zu einer Reduktion der Aß-Produktion führen könnte, zum anderen eine Aktivierung der α-Sekretase die Prozessierung von APP in APPsec steigern und damit gleichzeitig die Entstehung des Aß-Peptids reduzieren würde.

Es gibt viele Hinweise darauf, daß das Aß-Peptid bei dem Auftreten der Alzheimer'schen Krankheit ein entscheidender Faktor ist. Unter anderem wird eine Neurotoxizität von Aß-Fibrillen in Zellkultur postuliert (Yankner, B.A. et al., (1990) Proc Natl Acad Sci USA,87, 9020). Auch tritt bei Patienten mit Down Syndrom, bei denen APP in einer zusätzlichen Kopie vorkommt die für den Morbus Alzheimer charakteristische Neuropathologie bereits im Alter von 30 Jahren auf. Dabei wird angenommen, daß der Überexpression von APP eine erhöhte Umsetzung in das Aß-Peptid folgt (Rumble, B. et al., (1989), N. Engl. J. Med., 320, 1446).

Den vielleicht stärksten Hinweis auf die zentrale Rolle des Aß-Peptids stellen die familiären Formen der Alzheimer'schen Krankheit dar. Hier finden sich Mutationen im APP-Gen um den Bereich der ß- und γ-Sekretaseschnittstellen oder in zwei weiteren AD-assoziierten Genen (Presenilinen), die in Zellkultur zu einer wesentlichen Erhöhung der Aß-Produktion führen (Scheuner, D. et al., (1996), Nature Medicine, 2, 864).

C. elegans wurde bereits als Modellorganismus bei der Alzheimer'schen Krankheit eingesetzt, jedoch beziehen sich diese Arbeiten nicht auf die Prozessierung von APP in das Aß-Peptid. Einige Arbeiten befassen sich mit zwei anderen Alzheimer assoziierten Proteinen, den Presenilinen. Bei den Presenilinen handelt es sich um Transmembranproteine, die die Membran 6 - 8 mal durchqueren. Sie besitzen bei familiären Alzheimerfällen eine große Bedeutung, da bestimmte Mutationen in den Presenilingenen zur Alzheimer'schen Krankheit führen. Dabei wurde gezeigt, daß im C. elegans Homologe zu den humanen Presenilinen (sel-12, spe-4, hop-1) existieren, wobei die Funktion der Preseniline in Mensch und Wurm konserviert ist (Levitan D, Greenwald I (1995) Nature 377, 351; Levitan et al.(1996) Proc Natl Acad Sci USA, 93, 14940; Baumeister R (1997) Genes & Function 1, 149; Xiajun Li and Iva Greenwald (1997) Proc Natl Acad Sci USA, 94, 12204).

Weitere Arbeiten beschäftigen sich mit dem APP-Homologen in C. elegans, das als Apl-1 bezeichnet wird und der Aß-Peptid Expression in C. elegans. Apl-1 besitzt jedoch keine Region, die zu der Aminosäuresequenz des Aß-Peptids homolog ist, weshalb C. elegans kein endogenes Aß-Peptid hat (Daigle I, Li C (1993) Proc Natl Acad Sci USA, 90 (24), 12045).

Die Expression von Aß-Peptid (nicht die eines Aß-Vorläuferproteins) in C. elegans wurde von C. D. Link, Proc Natl Acad Sci USA (1995) 92, 9368 beschrieben. In diesen Arbeiten wurden transgene Würmer hergestellt, die ein Aß1-42 Peptid (d.h. das Aß-Peptid, das aus 42 Aminosäuren besteht) unter der Kontrolle des Muskelspezifischen Promotors unc 54 als Fusionsprotein mit einem synthetischen Signalpeptid exprimiert. In diesen Studien wurden Muskel-spezifische Proteinablagerungen, die mit anti-ß-amyloid Antikörpern reagierten, nachgewiesen. Weitere Arbeiten (z.B. C. Link et al. persönliche Mitteilung) beziehen sich auf Untersuchungen der Aggregation und Toxizität des Aß-Peptids im Modellsystem C. elegans.

Um die Existenz einer Prozessierungsmaschinerie in C. elegans, die an der Entstehung von Aß-Peptid beteiligt ist, zu untersuchen und potentielle Sekretasen im Wurm zu identifizieren wurden in der vorliegenden Arbeit transgene C. elegans Linien etabliert.

Gegenstand der vorliegenden Erfindung betrifft daher folgende Verwendung.

Verwendung eines transgenen C. elegans enthaltend ein Transgen enthaltend
a) eine Nukleotidsequenz, die für das Amyloid Vorläufer Protein (APP) oder einen Teil davon kodiert,
   wobei die Nukleotidsequenz den Teil der Nukleotidsequenz des APPs umfasst, der für das vollständige Aβ-Peptid kodiert und
   wobei die Nukleotidsequenz nicht identisch ist mit dem Teil des APPs, der für ein vollständiges Aß-Peptid kodiert,
b) gegebenenfalls ein oder mehrere weitere kodierende und/oder nicht-kodierende Nukleotidsequenzen und
c) einen Promotor für die Expression in einer Zelle des Nematoden Caenorhabditis elegans (C. elegans)
   zur Identifizierung einer γ-Sekretase Aktivität und/oder einer α-Sekretase Aktivität in C. elegans.

Vorzugsweise kodiert die Nukleotidsequenz für die 100 carboxy-terminalen Aminosäuren von APP, beginnend mit der Sequenz des Aß-Peptids und endend mit der carboxy-terminalen Aminosäure von APP, kodiert (C100 Fragment). Das APP ist vorzugsweise eines der Isoformen APP695 (695 Aminosäuren), APP751 (751 Aminosäuren), APP770 (770 Aminosäuren) und L-APP. Alle Isoformen entstehen durch alternatives Spleißen aus dem gleichen APP Gen. In APP695 wurden durch Spleißen die Exons 7 und 8 entfernt, wohingegen in APP751 nur das Exon 8 fehlt und in APP770 Exon 7 und 8 vorliegen. Daneben existieren weitere Spleißformen von APP, in denen das Exon 15 durch Spleißen entfernt wird. Diese Formen werden als L-APP bezeichnet und liegen ebenfalls in den bzgl. der Exons 7 und 8 gespleißten Formen vor.

In einer besonderen Ausführungsform der Erfindung enthält das Transgen die Nukleotidsequenz SEQ ID NO.: 1 oder einen Teil davon.

Vorzugsweise enthält das Transgen eine weitere kodierende Nukleotidsequenz, die am 5'-Ende der Nukleotidsequenz, die für APP oder einen Teil desselben kodiert, lokalisiert ist. In einer besonderen Ausführungsform der Erfindung kodiert die weitere Nukleotidsequenz für ein Signalpeptid oder einen Teil davon, beispielsweise für das APP Signalpeptid (SP) mit der Aminosäuresequenz SEQ ID NO.: 9 oder einen Teil davon.

Die Sequenz vom N-Terminus des Aß-Peptids bis zum C-Terminus von APP besteht aus 99 Aminosäuren. Das APP-Signalpeptid besteht aus 17 Aminosäuren. Bei der Klonierung eines Fusionsproduktes bestehend aus dem N-Terminus des Aß-Peptids bis zum C-Terminus von APP und dem APP Signalpeptid, werden zwischen diese beiden Teile des Fusionsproduktes vorzugsweise ein oder mehrere "Spacer-Aminosäuren" eingefügt, vorzugsweise wird eine Aminosäure eingefügt, beispielsweise Leucin. Das C-Terminale Fragment wird deshalb unterschiedlich bezeichnet, z.B. C100 (C= C-Terminus), LC99 (L=Leucin), LC1-99, C99, SPA4CT (SP=Signalpeptid, A4=Aß-Peptid, CT= C-Terminus).

In einer besonderen Ausführungsfrom der Erfindung enthält das Transgen die Nukleotidsequenz SEQ ID NO.: 2 oder einen Teil davon und/oder die Nukleotidsequenz SEQ ID NO.: 3 oder eine Teil davon.

Darüber hinaus kann das Transgen eine oder mehrere weitere nicht-kodierende und/oder eine oder mehrere weitere kodierende Nukleotidsequenzen enthalten. Beispielsweise kann das Transgen als weitere nicht-kodierende Nukleotidsequenz eine Sequenz aus einem Intron des APP Gens enthalten, z.B. eine Sequenz, die aus dem Intron 42 bp des APP Gens stammt und die Sequenz SEQ ID NO.: 4 aufweist. Gegenstand der Erfinndung ist ein Transgen, das die Nukleotidsequenz SEQ ID NO.: 5 enthält.

Vorzugsweise enthält das Transgen auch ein oder mehrere genregulatorische Sequenzen für die geregelte Expression des kodierten Proteins, vorzugsweise einen konstitutiven oder einen regulierbaren Promotor. Beispielsweise kann der Promotor in neuronalem, muskulärem oder dermalem Gewebe von C. elegans aktive sein oder ubiquitär in C. elegans aktiv sein. Ein Promotor kann beispielsweise ausgewählt werden aus der Reihe der C. elegans Promotoren unc-54, hsp16-2, unc-119, goa-1 und sel-12. In einer besonderen Ausführungsform der Erfindung enthält das Transgen einen Promotor mit der Nukleotidsequenz SEQ ID NO.: 6. In einer besonderen Ausführungsform enthält das Transgen die Nukleotidsequenz SEQ ID NO.: 7.

Das Transgen kann in einem Vektor vorliegen, beispielsweise in einem Expressionsvektor. Beispielsweise kann ein rekombinanter Expressionsvektor die Nukleotidsequenz SEQ ID NO.: 8 enthalten.

Die Erfindung beschreibt auch die Herstellung eines Expressionsvektors, wobei ein Transgen nach bekannten Methoden in einen Vektor integriert wird. Insbesondere beschreibt die Erfindung die Verwendung eines Expressionsvektors zur Herstellung einer transgenen Zelle, wobei diese Zelle Teil eines nicht-humanen Organismus, z.B. C. elegans, sein kann.

Die Erfindung beschreibt auch die Herstellung des Transgens, wobei geeignete Teilsequenzen in der entsprechenden Reihenfolge und im richtigen Leserahmen, ggf. unter Einfügung von Linkern ligiert werden. Insbesondere beschreibt die Erfindung die Verwendung des Transgens, z.B. zur Herstellung einer transgenen Zelle, wobei diese Zelle Teil eines nicht humanen Organismus sein kann. Beispielsweise kann die Zelle eine C. elegans Zelle sein.

Eine besondere Ausführungsform der Erfindung beschreibt einen transgenen C. elegans, der das Transgen enthält. Das Transgen kann in dem C. elegans auch in einem Expressionsvektor vorliegen. Das Transgen kann in dem C. elegans intra-und/oder extrachromosomal vorliegen. Intra- und/oder extrachromosomal können ein oder mehrere Transgene bzw. Expressionsvektoren, die das Transgen enthalten, als

Long Tandem Arrays vorliegen. Vorzugsweise enthält eine transgene Zelle oder ein transgener Organismus zusätzlich einen weiteren Expressionsvektor, der eine Nukleotidsequenz enthält, die für einen Marker kodiert, wobei der Marker entweder ein temperatursensitiver oder ein phenotypischer Marker ist. Beispielsweise kann der Marker ein visueller oder ein verhaltens-phenotypischer Marker sein. Beispiele sind fluoreszierende Marker, z.B. GFP (green fluorescent protein) oder EGFP (Enhanced green fluorescent protein), Markergene, die für eine dominante mutierte Form eines bestimmten Proteins kodiert, z.B. für eine dominante RoI6 Mutation oder Markersequenzen, die für antisense RNA kodieren, z.B. für die antisense RNA von Unc-22 ist.

Vorzugsweise liegen in den Keimzellen und/oder den somatischen Zellen des transgenen C. elegans eine oder mehrere Kopien des Transgens und/oder des Expressionsvektors vor und ggf. eines weiteren Expressionsvektor vor.

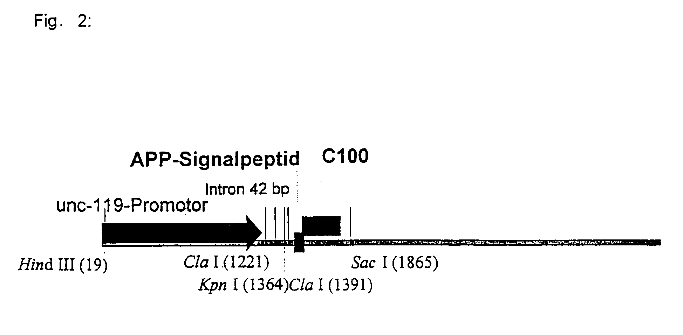

Die Erfindung beschreibt auch Verfahren zur Herstellung eines transgenen C. elegans, wobei ein Transgen und/oder ein Expressionsvektor gegebenenfalls in Gegenwart eines weiteren Expressionsvektors, der eine Nukleotidsequenz enthält, die für einen Marker kodiert, in die Keimzellen eines C. elegans mikroinjiziert wird. Für die Herstellung der transgenen C. elegans-Linien kann beispielsweise ein DNA-Konstrukt verwendet, das SP-C100 (SP = Signalpeptid) unter der Kontrolle eines Neuron-spezifischen Promotors exprimiert (Abb. 2). Da sich C100 aus der Aβ-Sequenz und dem C-Terminus von APP zusammensetzt ist nur der γ-Sekretaseschnitt nötig, um das Aß-Peptid aus C100 freizusetzten. C100 stellt ebenfalls ein Substrat für die γ-Sekretase dar.

Die Erfindung beschreibt auch die Verwendung eines transgenen C. elegans, beispielsweise zur Expression eines SP-C100 Fusionsproteins. Ein Gegenstand der Erfindung verwendet ein Sp-C100 Fusionsprotein mit der Aminosäuresequenz SEQ ID NO.: 10.

Insbesondere betrifft die Erfindung die Verwendung eines transgenen C. elegans zur Identifizierung einer γ-Sekretase Aktivität und/oder einer α-Sekretase Aktivität in C. elegans, die Verwendung in Verfahren zur Identifizierung und/oder Charakterisierung von Substanzen, die die γ-Sekretase Aktivität hemmen, die Verwendung in Verfahren zur Identifizierung und/oder Charakterisierung von Substanzen, die die α-Sekretase Aktivität erhöhen, die Verwendung in Verfahren zur Identifizierung und/oder Charakterisierung von Substanzen, die als Wirkstoffe zur Behandlung und/oder Prävention der Alzheimerschen' Krankheit verwendet werden können.

Zur Identifizierung von Sekretasen, die an der Prozessierung von APP in das Aβ-Peptid beteiligt sind wurde in der vorliegenden Arbeit der Nematode Caenorhabditis elegans (C. elegans) als Modellorganismus gewählt. Dieser Wurm eignet sich hervorragend für genetische Studien und wurde daher in der Vergangenheit vielfach eingesetzt, um universell wichtige Prozesse wie z.B. den programmierten Zelltod, Neuronal guidance und RAS/MAP Kinase Signalling zu untersuchen (Riddle, D.L. et al. (1997)).

Zu den wesentlichen Punkten, durch die sich C. elegans besonders für solche Studien auszeichnet, zählen (C. Kenyon, Science (1988) 240, 1448; P.E. Kuwabara (1997), TIG, 13, 454):
- sein kleines Genom, das sich aus etwa 19000 Genen bzw. 97 Mb zusammensetzt und im Dezember 1998 vollständig sequenziert worden ist. (The C. elegans Sequencing Consortium, Science (1998), 282, 2012).
- seine Vermehrung durch Selbstbefruchtung. Bei den zwei Geschlechtern von C. elegans unterscheidet man zwischen Männchen und Hermaphroditen, d.h. zwittrige Tiere, die ihre Eier vor der Ablage selbst befruchten. Ein entscheidender Vorteil dieser Art der Vermehrung ist, daß ein Hermaphrodit nach dem Einbringen eines Transgens in die Keimbahn automatisch homozygote transgene Nachkommen erzeugen kann. Es sind also keine weiteren Kreuzungsschritte wie z.B. bei Drosophila zur Herstellung transgener Linien notwendig.
- seine leichte Handhabung im Labor aufgrund seiner geringen Größe (etwa 1 mm Länge) und seiner relativ anspruchslosen Wachstumsbedingungen. Es kann daher eine große Zahl von Würmern routinemäßig im Labor gehandhabt werden.
- seine kurze Generationszeit von 3 Tagen, die es ermöglicht innerhalb sehr kurzer Zeit große Mengen an biologischem Material für Analysen zu erhalten.
- Es ist eine komplette Zellbeschreibung für die Entwicklung und Anatomie von C. elegans vorhanden.
- Es liegen detaillierte genetische Karten und Methoden zur genetischen Analyse in C. elegans vor.
- Es sind Technologien zur Herstellung von knock-out Tieren vorhanden. Ebenso existieren Technologien zur Mutagenisierung des C. elegans Genoms (Transposon Mutagenese, Ethylmethansulfonate (EMS) Mutagenese).

Verwendungsmöglichkeiten der transgenen C. elegans Linien:
1. Identifizierung einer γ-Sekretase ähnlichen Aktivität in C. elegans mit Hilfe von Mutageneseansätzen. Geplant ist eine Transposonmutagenese, durch die die γ-Sekretase ähnliche Aktivität zerstört und durch Detektion der Würmer, die diese Aktivität nicht mehr besitzen, das entsprechende Gen gesucht werden soll. Solch ein Screeningverfahren ist in der Literatur beschrieben: Korswagen H.C. et al., (1996), 93, 14680 Proc Natl Acad Sci USA).
   Alternative Ansätze wären Mutagenese über Ethylmethansulfonat (EMS) oder auch RNA anti-sense Ansätze. Bei letzterem könnte man versuchen gemeinsame Motive aller C. elegans Proteasen zu finden und gezielt mit anti-sense RNA, die gegen diese Motive gerichtet sind, herunter zu regulieren. Ein Screening auf das Aß-Peptid könnte dann zeigen, ob eine der Proteasen an der Aß-Produktion beteiligt ist.
2. Identifizierung einer γ-Sekretase ähnlichen Aktivität in C. elegans evtl. über einen ähnlichen Weg wie in Punkt 1. beschrieben.
3. Mit der Kenntnis einer γ-Sekretase bzw. γ-Sekretase ähnlichen Aktivität in C. elegans kann über Homologievergleich die humane γ-Sekretase bzw. γ-Sekretase ähnliche Aktivität gesucht werden.
4. Identifizierung von Pharmaka, die
   - die γ-Sekretaseaktivität hemmen, um direkt die Aß-Produktion aus dem Amyloidvorläuferprotein zu inhibieren.
   - die γ-Sekretase aktivieren und dadurch indirekt durch eine Steigerung der APPsec-Produktion die Entstehung des Aß-Peptids inhibieren.
      Dieser Ansatz könnte im 96-Well-Format stattfinden, da C. elegans in Suspension in 96-Well-Platten gehalten werden kann.
      Da das Screening an einem Gesamtorganismus durchgeführt wird, können Pharmaka mit unspezifisch toxischer Wirkung weitgehend ausgeschlossen werden.
5. Untersuchung des Aggregationsverhaltens und einer eventuellen neurotoxischen Wirkung des Aß-Peptids in C. elegans. Screening nach Pharmaka, die die Aggregation von Aß-Peptid inhibieren.
6. Untersuchung zur Modulation der APP-Prozessierung durch andere Proteine (z.B. Preseniline oder ApoE) durch deren Überexpression oder knock-out. Da es sich bei den Presenilinen um Alzheimer-assoziierte Proteine handelt und ApoE ein Risikofaktor der Alzheimer Krankheit darstellt könnten diese Proteine die Bildung des Aß-Peptids beeinflussen und damit ihre Rolle im APP-Prozessierungsweg untersucht werden.
7. Gegebenenfalls Validierung einer α- und/oder γ-Sekretaseaktivität, die mit Hilfe anderer, dem Fachmann bekannter, experimenteller Ansätze gefunden wurde.

Figur 1: Figur 1 zeigt das Amyloid Vorläuferprotein (Isoform APP695 und Isoformen APP770 bzw. APP751) und Sekretase Spaltprodukte.

Figur 2: Figur 2 beschreibt die Konstruktion des transgenen Vektors "Unc-119-SP-C100" enthaltend einem unc-119 Promotor, ein APP-Signalpeptid und das C100 Fragment aus APP, wobei "unc-119" ein Neuron-spezifischer C. elegans Promotor ist, APP Signalpeptid, den Aminosäuren 1 bis 24 von APP entspricht und C100 den C-terminalen 100 Aminosäuren von APP (= C100) entspricht. C100 besteht aus der Aß-Sequenz und dem C-Terminus von APP (Shoji, M et al., (1992) Science 258, 126). Der Vektor Unc-119-SP-C100 hat 5112 Basenpaare.

### Beispiele:

### Beipiel 1: Herstellung eines Expressionsvektors, der das Transgen enthält.

Für die Klonierung wurden zwei Vektoren verwendet, pSKLC1-99, der das SP-C100 kodiert und pBY103, der den unc-119 Promotor enthält, wobei die für SP-C100 kodierende DNA hinter den unc-119 Promotor in den pBY103-Vektor kloniert wurde. Der Basisvektor pBY103 besteht aus dem Vektorbackbone pPD49.26, beschrieben in "Caenorhabditis elegans: Modern Biological Analysis of an Organism" (1995) Ed. Epstein et al., Vol 48, pp. 473 in das der unc-119 Promotor (Maduro et al. Genetics (1995), 141, p. 977) über HindIII/BamHI kloniert wurde. Das Plasmid unc-119-SP-C100 wurde durch KpnI/SacI Schnitt und anschließende Klonierung des LC99 Fragments aus pSKLC1-99 (Shoji et al. (1992) in den pBY103 hergestellt.

### Beispiel 2: Herstellung der transgenen C. elegans Linien

Zur Herstellung der transgenen C. elegans Linien wurde das Verfahren der Mikroinjektion angewandt (Mello et al.,(1991) EMBO J. 10 (12) 3959; C. Mello and A. Fire, Methods in Cell Biology, Academic Press Vol48, pp451, 1995; C. D. Link, Proc Natl Acad Sci USA (1995) 92, 9368).

Verwendet wurden zwei verschiedene C. elegans-Stämme, Wild-typ N2 und him-8 (high incidence of males). Das unc-119-SP-C100-Konstrukt wurde in die Gonaden von jungen adulten Hermaphroditen mit Hilfe einer Mikroinjektionsanlage mikroinjiziert. Die DNA-Konzentration betrug etwa 20 ng/µl.

Zusammen mit dem unc-119-SP-C100 wurde ein Markerplasmid injiziert. Dabei handelt es sich um das ttx3-GFP-Plasmid, das für das Green Fluorescent Protein unter der Kontrolle des ttx3-Promotors kodiert. Die Aktivität des ttx3-Promotors ist spezifisch für bestimmte Kopfneuronen von C. elegans, den sogenannten AIY-Neuronen, die bei der Thermotaxis des Wurms eine Rolle spielen.

Bei der Mikroinjektion von Plasmid-DNA wird davon ausgegangen, daß sich durch Rekombination sogenannte Long Tandem Arrays bilden, die aus vielen Kopien von Plasmid-DNA (in unserem Falle aus dem ttx3-GFP und dem unc-119-SP-C100) bestehen. Diese Arrays integrieren zu einem bestimmten Prozentsatz in das C. elegans Genom. Mit einer höheren Wahrscheinlichkeit jedoch liegen die Arrays extrachromosomal vor.

Erfolgreich injizierte Würmer weisen bei Anregung mit Licht einer Wellenlänge von etwa 480 nm grüne Fluoreszenz in den AIY-Neuronen im Kopfbereich auf. Solche Nematoden konnten detektiert werden.

### Beispiel 3: Beschreibung der C100 transgenen C. elegans Linien

### 1. Phänotypische Merkmale

C100 transgene Würmer weisen in den AIY-Neuronen des Kopfbereiches nach Anregung mit Licht der Wellenlänge von 480 nm eine grüne Fluoreszenz auf. Da auch bei den Nachkommen der Würmer wiederum grüne Fluoreszenz in den Kopfneuronen detektierbar war, kann davon ausgegangen werden, daß die Plasmide keimbahngängig sind. Jedoch ist die Penetranz nicht 100 %, was darauf schließen läßt, daß die long tandam arrays aus ttx3-GFP-Marker-DNA und unc-119-SP-C100 eher extrachromosomal als in das Genom integriert vorliegen.

### Beispiel 4: Nachweis der C100 Expression im Blot

Sechs verschiedene transgene C100 C. elegans Linien (drei im N2 wt und drei im him 8 Hintergrund) wurden in einem Western Blot auf die Expression des C100 Fragments mit einem polyklonalen Antiserum, das gegen den C-Terminus von APP gerichtet ist, untersucht. In allen sechs Linien war eine Bande mit dem entsprechenden Molekulargewicht von etwa 10 kDa detektierbar.

### Beispiel 5: Nachweis des C100 im ELISA

In einem Aβ Sandwich ELISA konnten in Zellextrakten aus transgenen Tieren Signale über dem Hintergrund-Level detektiert werden, die in zwei Fällen statistisch signifikant waren. Dies deutet darauf hin, daß C. elegans eine γ-Sekretase ähnliche Aktivität besitzen könnte.

Bei dem Aß-Sandwich ELISA Assay werden 96-Well-Platten zunächst mit dem monoklonalen Antikörper Klon 6E10 (SENETEK PLC., MO, USA), der spezifisch mit dem Aß-Peptid (Aminosäure 1-17) reagiert, inkubiert und anschließend mit Wurmextrakten aus transgenen Würmern bzw. Kontrollwürmern überschichtet. Die Detektion des Aß-Peptids findet mit Hilfe des monoklonalen Aß-Antikörper 4G8 (SENETEK PLC., MO, USA), der die Aminosäuren 17-24 im Aß-Peptid erkennt und mit Biotin markiert ist, statt. Der Nachweis erfolgt über die Alkalische Phosphatase Reaktion mit einem entsprechenden Antikörper der gegen Biotin gerichtet ist. Der Aufschluß der Würmer impliziert Detergenzbehandlung, Stickstoffschockgefrieren, Sonifizierung, und das Aufbrechen der Zellen mit Glasperlen.

Das ELISA Signal aus dem oben beschriebenen Versuch kann sowohl einer schwachen Expression des Aß-Peptids als auch der des C100-Vorläuferproteins zugrunde liegen, da die entsprechenden Epitope in beiden Proteinen vorliegen.

In analoger Weise könnte beispielsweise auch die Expression des Aß-Peptides spezifisch nachgewiesen werden: Dazu müßten Aß-spezifische Antikörper, die nicht mit dem C 100-Vorläufer reagieren, in einem Aß-Sandwich ELISA eingesetzt werden. Ein Aß-spezifischer Antikörper könnte beispielsweise ein monoklonaler Antikörper sein, der spezifisch das C-terminale Ende der Aß-Form erkennt, die sich aus 40 oder aus 42 Aminosäuren zusammensetzt. Parallel könnte das Aß-Peptid im Western Blot mit Hilfe der monoklonalen Antikörper 4G8 und 6E10 detektiert und dann aufgrund seines Molekulargewichtes von 4kD von dem größeren C100-Vorläufer unterschieden werden.

Die Vektoren sind erhältlich bei Andrew Fire (Department of Embryology, Carnegie Institution of Washington, Baltimore, Maryland 21210, USA) für den pPD49.26 bzw. LC99 (Amyloid Precursor protein) hinterlegt unter der ATCC-Nummer 106372. Der unc-119-Promotor ist erhältlich von Maduro, M. (Department of Biological Science, Universitiy of Alberta Edmonton, Canada), unc-54 und unc-16.2 sind von Andrew Fire erhältlich.
SEQ ID NO.1: Nukleotidsequenz von C100
SEQ ID NO.2: Nukleotidsequenz von SP
   ATG CTGCCCGGTT TGGCACTGTT CCTGCTGGCC GCCTGGACGG CTCGGGCG
SEQ ID NO.3: Nukleotidsequenz von SP+C100
SEQ ID NO.4: Nukleotidsequenz Intron 42bp
   GTATGTTTCGAATGATACTAACATAACATAGAACATTTTCAG
SEQ ID NO.5: Nukleotidsequenz von Intron+SP+C100
SEQ ID NO.6: Nukleotidsequenz von unc-119
SEQ ID NO.7: Nukleotidsequenz von unc-119+ Intron+SP+C100
SEQ ID NO.8: Nukleotidsequenz des Expressionsvektors
SEQ ID NO.9: Aminosäuresequenz von SP
   MLPGLALFLL AAWTARA
SEQ ID NO.10: Aminosäuresequenz des Fusionsproteins
   MLPGLALFLL AAWTARALDA EFRHDSGYEV HHQKLVFFAE DVGSNKGAII
   GLMVGGVVIA TVIVITLVML KKKQYTSIHH G\/VEVDAAVT PEERHLSKMQ
   QNGYENPTYK FFEQMQN
SEQ ID NO. 11: Nukleotidsequenz des Vektors unc-119-SP-C100

### Literatur:

- Baumeister R (1997) Genes & Function 1, 149
- Daigle I, Li C (1993), 90 (24), 12045
- Kang, J., Lemaire, H.G., Unterbeck, A., Salbaum J.M., Masters C.L., Grzeschik, K.H., Multhaupt, G., Beyreuther, K., Mueller-Hill, B. (1987) Nature, 325, 733
- Kenyon, C., Science (1988) 240, 1448
- Korswagen H.C., Durbin, R. M., Smits, M. T., Plasterk, R. H. A. (1996), 93, 14680 Proc Natl Acad Sci USA
- Kuwabara, P. E. (1997), Trends in Gentics, 13, 454
- Levitan D., Doyle TG, Brousseau D., Lee MK. Thinakaran G., Slunt HH., Sisodia SS. Greenwald I. (1996) Proc Natl Acad Sci USA, 93, 14940
- Levitan D, Greenwald I (1995) Nature 377, 351
- Link C.D. (1995) Proc Natl Acad Sci USA, 92, 9368
- Mello, C. and Fire, A., Methods in Cell Biology, Academic Press Vol.48, pp 451, 1995
- Riddle et al. (1997) C. elegans II, Cold Spring Habor Laboratory Press
- Rumble, B., Retallack, R., Hilbich, C., Simms, G., Multhaup, G., Martins, R., Hockey, A., Montgomery, P., Beyreuther, K., Masters, C.L., (1989), N. Engl. J. Med., 320, 1446
- Scheuner, D., Eckman, C., Jensen, M., Song, X., Citron, M., Suzuki, N., Bird, T., Hardy, M., Hutton, W., Kukull, W., Farson, E., Levy-Lahad, E., Vitanen, M., Peskind, E., Poorkaj, P., Schellenberg, G., Tanzi, R., Wasco, W., Lannfeld, D., Selkoe, D., Younkin, S.G. (1996), Nature Medicine, 2, 864
- Shoji M., Golde T E., Ghiso J., Cheung T T., Estus S., Shaffer L M., Cai X-D., McKay D M., Tintner R., Fraggione B., Younkin S G. (1992) Science 258, 126
- Xiajun Li and Iva Greenwald (1997) Proc Natl Acad Sci USA, 94, 12204
- Yankner, B.A., Caceres, A., Duffy, L.K. (1990) Proc Natl Acad Sci USA,87, 9020

## Patentansprüche

1. Verwendung eines transgenen C. elegans enthaltend ein Transgen enthaltend
a) eine Nukleotidsequenz, die für das Amyloid Vorläufer Protein (APP) oder einen Teil davon kodiert,
wobei die Nukleotidsequenz den Teil der Nukleotidsequenz des APPs umfasst, der für das vollständige Aß-Peptid kodiert und
wobei die Nukleotidsequenz nicht identisch ist mit dem Teil des APPs, der für ein vollständiges Aß-Peptid kodiert,
b) gegebenenfalls ein oder mehrere weitere kodierende und/oder nicht-kodierende Nukleotidsequenzen und
c) einen Promotor für die Expression in einer Zelle des Nematoden Caenorhabditis elegans (C. elegans)
zur Identifizierung einer γ-Sekretase Aktivität und/oder einer α-Sekretase Aktivität in C. elegans.

2. Verwendung nach Anspruch 1, wobei die Nukleotidsequenz für die 100 carboxyterminalen Aminosäuren von APP, beginnend mit der Sequenz des Aß-Peptids und endend mit der carboxyterminalen Aminosäure von APP, kodiert (C100 Fragment).

3. Verwendung nach einem der Ansprüche 1 oder 2, wobei das Transgen die Nukleotidsequenz SEQ ID NO.: 1 enthält.

4. Verwendung nach einem der Ansprüche 1 bis 3, wobei das Transgen eine weitere kodierende Nukleotidsequenz, die am 5'-Ende der Nukleotidsequenz, die für APP oder einen Teil desselben kodiert, lokalisiert ist, enthält.

5. Verwendung nach Anspruch 4, wobei die weitere Nukleotidsequenz für ein Signalpeptid oder einen Teil davon kodiert.

6. Verwendung nach einem der Ansprüche 4 oder 5, wobei die weitere Nukleotidsequenz für das APP Signalpeptid (SP) mit der Aminosäuresequenz SEQ ID NO.: 9 kodiert.

7. Verwendung nach einem der Ansprüche 4 bis 6 enthaltend die Nukleotidsequenz SEQ ID NO.: 2.

8. Verwendung nach einem oder mehreren der Ansprüche 1 bis 7 enthaltend die Nukleotidsequenz SEQ ID NO.: 3.

9. Verwendung nach einem der Ansprüche 1 bis 8 enthaltend eine weitere nicht-kodierende Nukleotidsequenz.

10. Verwendung nach Anspruch 9, wobei die weitere nicht-kodierende Nukleotidsequenz aus dem Intron 42 bp des APP Gens stammt und die Sequenz SEQ ID NO.: 4 aufweist.

11. Verwendung nach einem der Ansprüche 9 oder 10 enthaltend die Nukleotidsequenz SEQ ID NO.: 5.

12. Verwendung nach einem der Ansprüche 1 bis 11 enthaltend einen konstitutiven oder einen regulierbaren Promotor.

13. Verwendung nach einem der Ansprüche 1 bis 12, wobei der Promotor in neuronalem, muskulärem, dermalem Gewebe von C. elegans oder ubiquitär in C. elegans aktiv ist.

14. Verwendung nach einem der Ansprüche 1 bis 13 enthaltend einen Promotor ausgewählt aus der Reihe der C. elegans Promotoren unc-54, hsp 16-2, unc-119, G₀A1 und sel-12.

## Claims

1. The use of a transgenic C. elegans which comprises a transgene which contains
a) a nucleotide sequence which encodes the amyloid precursor protein (APP) or a part thereof,
with the nucleotide sequence comprising the part of the APP nucleotide sequence which encodes the complete Aß peptide, and
with the nucleotide sequence not being identical to the part of the APP which encodes a complete Aß peptide,
b) where appropriate, one or more further coding and/or non-coding nucleotide sequences, and
c) a promoter for expression in a cell of the nematode Caenorhabditis elegans (C. elegans) for identifying a γ-secretase activity and/or an α-secretase activity in C. elegans.

2. The use as claimed in claim 1, wherein the nucleotide sequence encodes the 100 carboxyterminal amino acids of APP, beginning with the sequence of the Aß peptide and ending with the carboxy terminal amino acid of APP (C100 fragment).

3. The use as claimed in either of claims 1 and 2, wherein the transgene contains the nucleotide sequence SEQ ID NO.: 1.

4. The use as claimed in one of claims 1 to 3, wherein the transgene contains an additional coding nucleotide sequence which is located at the 5' end of the nucleotide sequence which encodes APP or a part thereof.

5. The use as claimed in claim 4, wherein the additional nucleotide sequence encodes a signal peptide or a part thereof.

6. The use as claimed in either of claims 4 and 5, wherein the additional nucleotide sequence encodes the APP signal peptide (SP) having the amino acid sequence SEQ ID NO.: 9.

7. The use as claimed in one of claims 4 to 6, which contains the nucleotide sequence SEQ ID NO.: 2.

8. The use as claimed in one or more of claims 1 to 7, which contains the nucleotide sequence SEQ ID NO.: 3.

9. The use as claimed in one of claims 1 to 8, which contains an additional noncoding nucleotide sequence.

10. The use as claimed in claim 9, wherein the additional non-coding nucleotide sequence is derived from the 42 bp intron of the APP gene and exhibits the sequence SEQ ID NO.: 4.

11. The use as claimed in either of claims 9 and 10, which contains the nucleotide sequence SEQ ID NO.: 5.

12. The use as claimed in one of claims 1 to 11, which contains a constitutive promoter or a promoter which can be regulated.

13. The use as claimed in one of claims 1 to 12, wherein the promoter is active in neuronal, muscular or dermal tissue of C. elegans or is ubiquitously active in C. elegans.

14. The use as claimed in one of claims 1 to 13, which contains a promoter which is selected from the group of the C. elegans promoters unc-54, hsp16-2, unc-119, G₀A1 and sel-12.

## Revendications

1. Utilisation d'un C. elegans transgénique contenant un transgène contenant :
a) une séquence nucléotidique qui code pour la protéine précurseur de l'amyloïde (APP) ou une partie de cette dernière, la séquence nucléotidique comprenant la partie de la séquence nucléotidique de l'APP qui code pour le peptide Aβ complet, et la séquence nucléotidique n'étant pas identique à la partie de l'APP qui code pour un peptide Aβ complet,
b) éventuellement, une ou plusieurs séquences nucléotidiques codantes et/ou non codantes supplémentaires, et
c) un promoteur pour l'expression dans une cellule du nématode Caenorhabditis elegans (C. elegans),
pour l'identification d'une activité de γ-sécrétase et/ou d'une activité d'α-sécrétase dans C. elegans.

2. Utilisation selon la revendication 1, pour laquelle la séquence nucléotidique code pour les 100 acides aminés carboxy-terminaux de l'APP, en commençant par la séquence du peptide Aβ et se terminant par l'acide aminé carboxy-terminal de l'APP (fragment C100).

3. Utilisation selon l'une des revendications 1 ou 2, pour laquelle le transgène contient la séquence nucléotidique SEQ ID N° 1.

4. Utilisation selon l'une des revendications 1 à 3, pour laquelle le transgène contient une séquence nucléotidique codante supplémentaire, qui est localisée à l'extrémité 5' de la séquence nucléotidique qui code pour l'APP ou une partie de cette dernière.

5. Utilisation selon la revendication 4, pour laquelle la séquence nucléotidique supplémentaire code pour un peptide signal ou une partie de ce dernier.

6. Utilisation selon l'une des revendications 4 ou 5, pour laquelle la séquence nucléotidique supplémentaire code pour le peptide signal (SP) de l'APP, ayant la séquence d'acides aminés SEQ ID N° 9.

7. Utilisation selon l'une des revendications 4 à 6, contenant la séquence nucléotidique SEQ ID N° 2.

8. Utilisation selon l'une ou plusieurs des revendications 1 à 7, contenant la séquence nucléotidique SEQ ID N° 3.

9. Utilisation selon l'une des revendications 1 à 8, contenant une séquence nucléotidique non codante supplémentaire.

10. Utilisation selon la revendication 9, pour laquelle la séquence nucléotidique non codante supplémentaire provient de l'intron de 42 pb du gène de l'APP, et contient la séquence SEQ ID N° 4.

11. Utilisation selon l'une des revendications 9 ou 10, contenant la séquence nucléotidique SEQ ID N° 5.

12. Utilisation selon l'une des revendications 1 à 11, contenant un promoteur constitutif ou un promoteur régulable.

13. Utilisation selon l'une des revendications 1 à 12, pour laquelle le promoteur est actif dans le tissu neuronal, musculaire, dermique de C. elegans, ou ubiquitairement dans C. elegans.

14. Utilisation selon l'une des revendications 1 à 13, contenant un promoteur choisi dans la série des promoteurs de C. elegans unc-54, hsp 16-2, unc-119, G₀A1 et sel-12.
